# EUROPÄISCHE PATENTSCHRIFT

(11) **EP 4 146 314 B1**
(45) Veröffentlichungstag und Bekanntmachung des Hinweises auf die Patenterteilung: **13.08.2025**
(21) Anmeldenummer: 21722185.2
(22) Anmeldetag: 27.04.2021
(51) Int. Cl.: A61M 16/04, A61B 1/267, A61B 1/005

(54) **INTUBATIONSHILFE ZUM EINFÜHREN EINES TUBUS IN DIE LUFTRÖHRE EINES PATIENTEN**
INTUBATION AID FOR INSERTING A TUBE INTO A PATIENT'S TRACHEA
DISPOSITIF D'ASSISTANCE D'INTUBATION POUR INTRODUIRE UN TUBE DANS LA TRACHÉE D'UN PATIENT

(30) Priorität: 06.05.2020 DE 102020205723
(43) Veröffentlichungstag der Anmeldung: 15.03.2023
(73) Patentinhaber: Kunz, Reiner, 14532 Kleinmachnow (DE)
(72) Erfinder: SCHMIDT, Michael, Halifax, Nova Scotia B3H4S9 (CA); KUNZ, Reiner, 14532 Kleinmachnow (DE)
(74) Vertreter: Weickmann & Weickmann PartmbB
(86) Internationale Anmeldenummer: PCT/EP2021/060902
(87) Internationale Veröffentlichungsnummer: WO 2021/224054

(56) Entgegenhaltungen:
- EP-A1- 1 281 414
- EP-A1- 3 335 754
- EP-A2- 1 224 904
- WO-A1-2014/019014
- WO-A2-2018/005776
- GB-A- 2 084 023
- GB-A- 2 341 102
- US-A- 5 279 610
- US-A- 5 607 386
- US-A1- 2012 078 055
- US-A1- 2013 255 671
- US-A1- 2014 246 014
- US-A1- 2014 275 772
- US-A1- 2015 096 556
- US-A1- 2018 064 895

## Beschreibung

Die vorliegende Erfindung betrifft eine Intubationshilfe zum Einführen eines Tubus in die Luftröhre eines Patienten.

Diagnostische und therapeutische Eingriffe an Menschen und Tieren werden häufig in Narkose mit Sicherstellung einer ausreichenden Atmung durch Einführen eines Beatmungsschlauches in die Luftröhre vorgenommen. In der Regel wird dabei der Kopf rekliniert, um bei einer möglichst geraden Ausrichtung von Mundhöhle/Rachen/Kehlkopf/Luftröhre die Einführung des Beatmungsschlauches zu erleichtern. Dabei wird mit einem Intubationsspatel die Zunge zur Seite gehalten und unter visueller Kontrolle der Tubus (Beatmungsschlauch) in die Luftröhre vorgeschoben und dort geblockt, um eine reversible atraumatische und luftdichte Fixation an der Schleimhaut der Luftröhre zu erreichen. So kann auch mit einem erhöhten Beatmungsdruck in der Luftröhre und der Lunge ein Kollaps der Lungenbläschen vermieden werden, auch um den Gasaustausch zu optimieren.

Hierzu wird oftmals der Tubus direkt vom Bediener gegriffen und in den Patienten eingeführt. Dabei besteht allerdings ein Verletzungsrisiko des Patienten, da niemals eine vollständig gerade Ausrichtung von Mundhöhle/Rachen/Kehlkopf/Luftröhre erreicht werden kann, sondern hier eher ein S-förmiger Verlauf vorliegt, und der Tubus lediglich passiv (das heißt durch Abgleiten an Körperstrukturen des Patienten) dazu eingerichtet ist, an die Anatomie des Patienten angepasst zu werden.

Es gibt Ansätze im Markt, den Beatmungstubus auf ein Bronchoskop aufzusetzen, um über die Mechanik des Bronchoskops den Tubus aktiv manipulieren zu können. Jedoch sind Bronchoskope zu unhandlich, da sie für eine Intubation zu lang und auch zu dünn sind, so dass sie den sehr starren Tubus oftmals nicht ausreichend deformieren können oder auch abknicken können. Ferner sind Bronchoskope sehr teuer, so dass sie, gerade im Rahmen der COVID-19-Pandemie, nicht in einer für die große Anzahl an durchzuführenden Intubationen benötigten Anzahl verfügbar gemacht werden können.

Als Stand der Technik sei auf die Druckschriften EP 3 335 754 A1, EP 1 224 904 A2, US 2015/096556 A1, US 2013/255671 A1, US 2014/275772 A1, US 2018/064895 A1, EP 1 281 414 A1, WO 2014/019014 A1, US 5 279 610 A, GB 2 084 023 A und GB 2 341 102 A verwiesen, welche jeweils Intubationshilfen und verwandte Techniken, Vorrichtungen und Verfahren betreffen.

Es ist daher die Aufgabe der vorliegenden Erfindung, eine Intubationshilfe bereitzustellen, welche eine sichere Intubation auch im Rahmen eines critical airway managements gewährleisten kann.

Diese Aufgabe wird in einem ersten erfindungsgemäßen Aspekt durch eine Intubationshilfe zum Einführen eines Tubus in die Luftröhre eines Patienten gelöst,
wobei die Intubationshilfe einen länglichen Grundkörper umfasst, welcher an seinem einen Ende mit einem Handgriff verbunden ist und an seinem anderen Ende ein freies Ende aufweist,
wobei die Intubationshilfe, insbesondere der Handgriff, mit einer Bedienungseinrichtung bereitgestellt ist,
wobei der Grundkörper dazu eingerichtet ist, auf eine Betätigung der Bedienungseinrichtung hin, in einem ersten Bereich des Grundkörpers gekrümmt zu werden,
wobei der Grundkörper ferner dazu eingerichtet ist, auf eine weitere Betätigung der Bedienungseinrichtung hin, in einem zweiten Bereich des Grundkörpers gekrümmt zu werden, welcher von dem ersten Bereich getrennt und dem freien Ende des Grundkörpers benachbart angeordnet ist, und
wobei der Grundkörper ferner eine Positionierungseinrichtung umfasst, welche dazu eingerichtet ist, eine translatorische Verlagerung eines auf die Intubationshilfe aufgesetzten Tubus in wenigstens einer Richtung zu begrenzen.

Eine "translatorische Verlagerung" sei hier als eine Bewegung parallel zu einer Grundkörper-Längsachse zu verstehen. In anderen Worten als eine Verlagerung des Tubus in und entgegen seiner Aufsteckrichtung auf die Intubationshilfe.

Die Positionierungseinrichtung kann beispielsweise als ein Anschlag ausgebildet sein, gegen welchen ein Ende des Tubus anliegen kann. Dabei kann der Anschlag insbesondere relativ zu dem Grundkörper derart verstellbar sein, dass die Positionierungseinrichtung auf verschiedene Tubuslängen einstellbar bzw. verstellbar ist. Bei der Verwendung von Bronchoskopen muss derzeit Klebeband zur Hilfe genommen werden, um den Tubus am Bronchoskop zu halten.

Ferner sei dieser Stelle erwähnt, dass "eine Betätigung der Bedienungseinrichtung" in diesem Aspekt der Erfindung bedeuten soll, dass der Grundkörper bzw. ein auf dem Grundkörper angeordneter Tubus nicht direkt an dem zu krümmenden Bereich (beispielsweise händisch) gebogen wird, sondern dass diese Krümmung in einer entfernten Weise stattfindet, zum Beispiel über einen Seilzug, welcher mit der Bedienungseinrichtung verbunden ist. Die Bedienungseinrichtung kann hier insbesondere dazu geeignet sein, durch einen Daumen eines Benutzers betätigt zu werden. Durch das Einstellen der krümmbaren Bereiche der erfindungsgemäßen Intubationshilfe kann der Grundkörper und damit der darauf angeordnete Tubus derart deformiert werden, dass diese auf die anatomische Situation des Patienten beim Intubieren angepasst werden können und so ein Verletzungsrisiko, insbesondere des Kehlkopfes mit den Stimmbändern, reduziert oder sogar vollständig vermieden werden kann.

Alternativ oder zusätzlich zu einer mechanischen Betätigung des ersten bzw. des zweiten zu krümmenden Bereichs über die Bedienungseinrichtung kann eine Eingabe eines Bedieners an der Bedienungseinrichtung auch elektronisch erfasst werden und an Stellmotoren ausgegeben werden, welche dem ersten Bereich bzw. dem zweiten Bereich zugeordnet sind und dazu eingerichtet sind, eine entsprechende Krümmung des ersten Bereichs bzw. des zweiten Bereichs hervorzurufen.

Zum verbesserten Einführen der Intubationshilfe in den Patienten bzw. des Tubus auf die Intubationshilfe kann zumindest der Grundkörper an seiner Außenseite mit einer Anti-Haftbeschichtung versehen sein.

**In** einer Weiterbildung der vorliegenden Erfindung kann die Positionierungseinrichtung eine Halteeinrichtung umfassen, welche dazu eingerichtet ist, mit einem Abschnitt des Tubus einzugreifen, um diesen, zumindest translatorisch, relativ zu dem Grundkörper zu sichern, wobei die Halteeinrichtung insbesondere als ein dem Handgriff benachbart angeordneter Konus, welcher mit einem Abschnitt des Tubus reibschlüssig in Eingriff tritt, und/oder als eine Verriegelungseinheit ausgebildet ist, welche mit dem Abschnitt des Tubus formschlüssig in Eingriff tritt. Dabei soll der Ausdruck "sichern" derart verstanden werden, dass die Halteeinrichtung den Tubus an dem Grundkörper hält, bis eine durch die Halteeinrichtung vorbestimmte Lösekraft in einer Abziehrichtung des Tubus von dem Grundkörper überschritten wird. Sowohl für eine reibschlüssige Sicherung als auch für eine formschlüssige Sicherung kann die Halteeinrichtung eine aktive Lösefunktionalität umfassen, welche die vorbestimmte Lösekraft zum Entfernen des Tubus von dem Grundkörper reduzieren kann. Dies kann beispielsweise durch ein Verringern des radialen Ausmaßes des oben erwähnten Konus und oder durch eine Verlagerung der oben erwähnten Verriegelungseinheit realisiert werden. **In** einer möglichen Ausführungsform der Halteeinrichtung kann ein Haken einen Endabschnitt des Tubus hintergreifen, um den Tubus an dem Grundkörper zu sichern. Selbstverständlich kann die Halteeinrichtung dabei für unterschiedliche Tubusdurchmesser geeignet sein.

Erfindungsgemäß umfasst der Grundkörper einen Kanal, welcher sich zumindest teilweise parallel zu einer Längsachse des Grundkörpers erstreckt, und welcher dazu geeignet ist, Fluid aufzunehmen und das Fluid zu einem dem Handgriff zugeordneten Ende des Kanals zu leiten. Ist beispielsweise ein Tubus auf den Grundkörper aufgesetzt, so kann der Tubus zusammen mit dem Grundkörper einen entsprechenden Absaugkanal ausbilden. Dabei kann es vorteilhaft sein, wenn der freie Querschnitt des Tubus, welcher zum Absaugen von Fluid verwendbar ist, durch den Grundkörper möglichst minimal ausgefüllt wird.

Beispielsweise die Lungenerkrankung COVID-19 (Corona) wird durch SARS-CoV-2 Viren verursacht. Diese befinden sich vorwiegend im Mund-/Nasen/Rachenraum und im Kehlkopf und werden von Mensch zu Mensch durch Tröpfcheninfektion und Aerosolbildung in der Atemluft übertragen. Tröpfchen entstehen beim Sprechen, Singen, Husten, Niesen oder vertiefter Atmung und sinken je nach Größe zu Boden. Aerosole jedoch können sich in geschlossenen Räumen über Stunden schwebend in der Raumluft verteilen, mit entsprechend langer Infektionsmöglichkeit. Da sich SARS-CoV-2 Viren in großer Menge in der Schleimhaut der oberen Luftwege der Infizierten finden und Corona-Patienten bereits mehrere Tage vor Auftreten erster Symptome Virenausscheider und damit infektiös sind, ist jederzeit eine Weitergabe der Erreger und der Erkrankung möglich und begründet die extreme Gefährlichkeit dieser Viruserkrankung. Mindestens 80% der Infizierten haben dabei einen asymptomatischen Verlauf oder nur geringe Beschwerden.

Bei einer Intubation oder einer Endoskopie von Nasen/Rachenraum oder einer Bronchoskopie bzw. Gastroskopie kann der Untersucher einen Sicherheitsabstand zur erkrankten Person nicht einhalten und ist, auch wegen der maximal forcierten Atmung der Patienten bei COVID-19 Erkrankten, gefährdet, das infektiöse Material (Bakterien/Viren) aufzunehmen und an derselben Krankheit zu erkranken. Um das medizinische Personal vor einer Virus-Infektion zu schützen, werden daher Schutzanzüge, Schutzbrillen, visierartige Sichtschutze und Handschuhe getragen, Patienten mit Einmalfolien abgedeckt und vereinzelt beim Intubieren der Oberkörper und die Kopfregion mit Polyacrylkästen überdeckt, die mit >100 L/min abgesaugt werden können, um eine Virusverbreitung zu unterbinden.

Somit kann eine kontinuierliche Absaugung der virusbeladenen Luft aus dem Mund-/Nasen-/Rachenraum, dem Kehlkopf und der Luftröhre des Patienten während des Intubationsvorgangs eine Kontamination der Raumluft im OP-Saal, und folglich ein Infektionsrisiko des behandelnden Personals, verringern oder verhindern.

Erfindungsgemäß ist hierfür mit dem dem Handgriff zugeordneten Ende des Kanals wahlweise verbindbar
eine Saugeinheit und ein Viren- und Bakterienfilter, welcher dazu geeignet ist, Viren und Bakterien aus dem von dem Patienten angesaugten Fluid zu filtern, oder
eine Gasquelle, insbesondere eine Sauerstoffquelle, welche dazu eingerichtet ist, Sauerstoff über den Kanal an den Patienten abzugeben. Zum Beispiel kann am Ende des Kanals ein Lippenventil angeordnet sein. Auch kann mit den Kanal eine Druckmesseinrichtung gekoppelt sein, um eine Ein- und Ausatmungsphase des Patienten erkennen zu können, so dass während der Einatmungsphase Sauerstoff in die Lunge des Patienten eingeleitet werden kann und in der Ausatmungsphase das Aerosol abgeleitet und gefiltert werden kann, welches der Patient ansonsten ausatmen würde. Dazu kann es vorteilhaft sein, dass die Schnittstelle zum Absaugen von Aerosol als von einer Schnittstelle zum Einleiten von Sauerstoff getrennt ausgebildet ist.

Erfindungsgemäß ist ferner eine Wandung des Grundkörpers, in Umfangsrichtung des länglichen Grundkörpers betrachtet, derart unterbrochen ausgebildet, dass ein sich in Längsrichtung des Grundkörpers erstreckender Spalt gebildet ist, welcher den Kanal mit einer den Grundkörper umgebenden Außenseite verbindet. Zum Beispiel kann sich somit ein, im Querschnitt, im Wesentlichen sichelförmiger Grundkörper ergeben. Durch diesen Zugang zu dem Kanal von der Außenseite über zumindest einen Großteil der Längserstreckung des Kanals kann die Reinigbarkeit des Kanals deutlich verbessert werden, da so insbesondere Reinigungsutensilien seitlich in den Kanal eingeführt werden können und/oder sterilisierendes Fluid seitlich in den Kanal eintreten kann. Neben dem oben beschriebenen Kanal zum Absaugen des Aerosols aus dem Patienten bzw. zum Einleiten von Gas, wie Sauerstoff, kann der Grundkörper natürlich weitere Kanäle umfassen, welche sich insbesondere lediglich an deren Längsenden zu einer Außenseite des Grundkörpers hin öffnen können. Beispielsweise kann ein solcher weiterer Kanal zum Einführen einer Biopsiezange geeignet sein.

Insbesondere kann der Grundkörper einen sich verjüngenden Abschnitt aufweisen, welcher dem freien Ende des Grundkörpers benachbart, insbesondere zwischen dem freien Ende des Grundkörpers und dem zweiten krümmbaren Bereich des Grundkörpers, angeordnet ist. Dieser sich verjüngende Abschnitt kann insbesondere so ausgebildet sein, dass er zu einem Innendurchmesser des Tubus und/oder zu einer Länge des Tubus passend ausgebildet ist, das heißt einen graduellen Übergang von einem dem freien Ende des Grundkörpers zugeordneten Abschnitt zu wenigstens einem Innendurchmesser des Tubus, insbesondere einem Außendurchmesser des Tubus, ermöglicht. Hierzu kann die oben bereits erwähnte Positionierungseinrichtung und/oder Halteeinrichtung des Grundkörpers derart verlagerbar sein, dass das der Positionierungseinrichtung und/oder Halteeinrichtung entgegengesetzte Ende des Tubus mit dem sich verjüngenden Abschnitt des Grundkörpers ausgerichtet werden kann.

Dieser allmähliche, das heißt im Wesentlichen nicht sprungartige, Übergang von dem freien Ende des Grundkörpers zu dem Tubus kann verhindern, dass sich die Intubationshilfe bzw. der Tubus an den Stimmbänder des Patienten verhakt und die Stimmbänder des Patienten beim Einführen des Tubus beschädigt werden. Beispielsweise kann ein Abstand des freien Endes des Grundkörpers zu dem sich konisch verjüngenden (bzw., aus dieser Richtung betrachtet, dem sich erweiternden) Abschnitt des Grundkörpers in etwa 5 cm betragen.

**In** einer Ausbildung der vorliegenden Erfindung kann sich der Kanal von dem dem Handgriff zugeordneten Ende bis zu dem sich verjüngenden Abschnitt des Grundkörpers erstrecken. Das heißt, der Kanal endet an dem sich verjüngenden Abschnitt des Grundkörpers bzw. läuft dort allmählich nach radial außen aus. Dies kann, bei auf dem Grundkörper angeordnetem Tubus, eine Absaugung von Aerosol direkt an dem distalen Ende des Tubus ermöglichen.

Vorteilhafterweise kann an dem freien Ende des Grundkörpers eine Bildgebungseinheit, insbesondere ein Bildsensor, angeordnet sein, welche dazu eingerichtet ist, Bilddaten aufzunehmen und diese weiterzuleiten. Durch die Videodokumentation des Intubationsvorganges kann das nachfolgende Abhorchen beider Lungenflügel mit dem Stethoskop entfallen. Außerdem erhält der Bediener visuelle Informationen über die Ausrichtung der Intubationshilfe bzw. des Tubus beim Intubieren. In Kombination mit den oben erwähnten Stellmotoren zur Krümmung des ersten Bereichs bzw. des zweiten Bereichs kann es denkbar sein, dass die Bildgebungseinheit die aufgenommenen Bilddaten an eine Steuereinheit ausgibt, welche die Bilddaten analysiert (zum Beispiel in Bezug auf einen größten Abstand der Bildgebungseinheit zu einer anatomischen Struktur des Patienten, was beispielsweise auf den weiteren Verlauf der Luftröhre des Patienten hindeuten kann) und darauf basierend eine automatisierte Steuerung der Stellmotoren (zum Beispiel eine Ausrichtung des freien Endes des Grundkörpers in Richtung dieses größten Abstands) durchführt. Somit könnte es möglich sein, dass sich die erfindungsgemäße Intubationshilfe autonom, das heißt ohne eine jeweilige Eingabe in die Bedienungseinrichtung der Intubationshilfe, deformiert, um sich an eine anatomische Situation des Patienten beim Intubieren anzupassen. Natürlich kann es dabei vorteilhaft sein, wenn die Steuereinheit eine Plausibilitätskontrolle für entsprechende vorzunehmende Ansteuerungen der Stellmotoren durchführt, um beispielsweise vermeiden zu können, dass die Intubationshilfe beim Einführen in die Stimmbänder des Patienten eine ungewünschte Deformation durchführt. Hierfür kann die Bildgebungseinheit als eine Stereo-Kamera ausgebildet sein, welche dazu eingerichtet ist, einen Bereich unter unterschiedlichen Winkeln aufzunehmen, oder die Bildgebungseinheit kann eine sonstige Einrichtung zur Abstandsmessung umfassen.

Ferner kann die Intubationshilfe mit einer Sensoreinrichtung ausgestattet sein, welche dazu geeignet ist, eine Position/Orientierung der Intubationshilfe und/oder eine Berührung der Intubationshilfe durch einen Benutzer zu erfassen, und entsprechende Signale auszugeben, so dass auf Grundlage entsprechender Signale von der Sensoreinrichtung die Intubationshilfe eingeschaltet bzw. ausgeschaltet werden kann.

An dem freien Ende des Grundkörpers kann eine Beleuchtungsvorrichtung angeordnet sein, welche dazu eingerichtet ist, Licht, insbesondere an den durch die Bildgebungseinheit aufzunehmenden Bereich, zu emittieren. Dabei kann eine Lichtquelle direkt an dem distalen Ende des Grundkörpers angeordnet sein oder das zu emittierende Licht kann von einer externen Lichtquelle in Lichtleiter eingespeist werden, über welche das Licht an dem distalen Ende des Grundkörpers ausgegeben werden kann.

Insbesondere kann der erste krümmbare Bereich in einem Abstand zu dem freien Ende des Grundkörpers von 10 cm bis 20 cm, insbesondere bei etwa 15 cm, angeordnet sein. Diese Anordnung des ersten Bereichs kann eine Anpassung des Grundkörpers bzw. des Tubus auf die Anatomie der Großzahl von Patienten ermöglichen.

Auch können Ebenen, in welchen der Grundkörper in dem ersten Bereich und in dem zweiten Bereich krümmbar sind, im Wesentlichen parallel zueinander, insbesondere zusammenfallend sein. Das bedeutet, dass die Krümmung des ersten Bereichs und die Krümmung des zweiten Bereichs in eine im Wesentlichen gleiche radiale Richtung und/oder in eine im Wesentlichen entgegengesetzte radiale Richtung verlaufen.

In einer vorteilhaften Ausbildung der vorliegenden Erfindung kann der erste Bereich von 0° bis 30° krümmbar sein und/oder der zweite Bereich von -60° bis 45° krümmbar sein. Im Rahmen der vorliegenden Erfindung soll hier 0° einer geraden Ausrichtung von Abschnitten des Grundkörpers vor und hinter dem jeweiligen krümmbaren Bereich sein, das heißt eine im Wesentlichen gerade Längserstreckung des Grundkörpers in Bezug auf den jeweiligen Bereich. Eine Krümmung des ersten Bereichs um 30° bezieht sich auf eine Auslenkung des Abschnitts des Grundkörpers, welcher distal von dem ersten Bereich angeordnet ist, relativ zu der 0°-Ausrichtung um 30°. Dabei können positive Gradzahlen (hier ohne Vorzeichen) als sich zu einer ersten Seite, insbesondere der kranialen Seite, erschreckend und negative Gradzahlen (zum Beispiel -60°) als sich zu einer zweiten Seite, insbesondere der caudalen Seite, erstreckend verstanden werden.

Die Bedienungseinrichtung kann wenigstens ein erstes Bedienungselement und ein zweites Bedienungselement umfassen, wobei das erste Bedienungselement für eine Auslenkung des ersten Bereichs des Grundkörpers eingerichtet ist und das zweite Bedienungselement für eine Auslenkung des zweiten Bereichs des Grundkörpers eingerichtet ist, und wobei dem ersten Bedienungselement eine Rasteinrichtung zugeordnet ist, welche dazu eingerichtet ist, auf eine Betätigung des ersten Bedienungselements um wenigstens ein vorbestimmtes Ausmaß hin, eine Rückwärtsbewegung des ersten Bedienungselements, entgegen der Betätigungsrichtung davon, zu verhindern. Die Rasteinrichtung kann natürlich lösbar ausgebildet sein, um das entsprechende Bedienungselement und damit den zugeordneten krümmbaren Bereich in seine ursprüngliche Ausrichtung zurück verlagern zu können. Alternativ oder zusätzlich kann die Rasteinrichtung auch durch eine Betätigung eines speziellen Bedienungselements im Handgriff, insbesondere an einer dort ausgebildeten Finger-Auflagefläche, aktiviert, das heißt der erste Bereich arretiert, werden. Dabei kann das zweite Bedienungselement insbesondere keine derartige Rasteinrichtung umfassen, so dass eine freie Vorwärts- und Rückwärtsbewegung des zweiten Bereichs des Grundkörpers gewährleistet bleiben kann.

Die Intubationshilfe kann ferner ein Kommunikationsmodul, insbesondere ein drahtloses Kommunikationsmodul, umfassen, welches dazu eingerichtet ist, mit einer externen Einheit zu kommunizieren, wobei das Kommunikationsmodul insbesondere in dem Handgriff der Intubationshilfe angeordnet ist. Ein drahtloses Kommunikationsmodul kann beispielsweise für eine WLAN-Kommunikation eine LiFi-Kommunikation oder eine Bluetooth-Kommunikation eingerichtet sein. Die externe Einheit kann eine Kommunikationsschnittstelle sein, welche mit einem Monitor verbunden oder integral zu diesem ist, um die durch die Bildgebungseinheit der Intubationshilfe aufgenommenen Bilddaten darzustellen.

Insbesondere im Falle einer drahtlosen Kommunikation der erfindungsgemäßen Intubationshilfe mit der externen Einheit können sowohl die Intubationshilfe als auch die externe Einheit, beispielsweise der Monitor, mit einer integrierten Stromversorgung versehen sein. Die integrierte Stromversorgung kann als Akkumulator ausgebildet sein, welcher zum Beispiel induktiv geladen werden kann.

Um eine Kommunikation der Intubationshilfe mit einem zugeordneten Monitor zu gewährleisten bzw. eine ungewünschte Kommunikation einer Intubationshilfe aus einem ersten Intubationshilfe-Monitor-Paar mit einem Monitor aus einem zweiten Intubationshilfe-Monitor-Paar zu verhindern, kann eine Kommunikation des Kommunikationsmoduls der Intubationshilfe und der zugeordneten externen Einheit über eine aktive Koppelung (zum Beispiel durch Betätigen entsprechender Tasten innerhalb eines vorbestimmten Zeitraums) verbunden werden.

Der Handgriff kann wenigstens einen Einfinger-Aufnahmeabschnitt, welcher dazu ausgebildet ist, einen Zeigefinger eines Bedieners aufzunehmen, und einen Zweifinger-Aufnahmeabschnitt umfassen, welcher dazu ausgebildet ist, einen Mittelfinger und einen Ringfinger eines Bedieners aufzunehmen, wobei sich der Einfinger-Aufnahmeabschnitt relativ zu dem Grundkörper an derjenigen Seite befinden kann, zu welcher hin der erste Bereich in seinem gekrümmten Zustand ausgelenkt ist. Diese Ausgestaltung des Handgriffs kann die Handhabbarkeit bei der üblichen Verwendungssituation der erfindungsgemäßen Intubationshilfe verbessern. So kann der Handgriff insbesondere derart relativ zu dem Grundkörper angeordnet sein, dass sich im Wesentlichen eine T-Form zwischen Handgriff und Grundkörper ergibt. Eine T-Form kann vorteilhaft sein, da in der OP-Einleitung der Patient auf dem OP-Tisch ungefähr in Hüfthöhe des Anästhesisten liegt. Im Notfalleinsatz kniet der Notarzt in der Regel vor dem Patienten, d.h. der Mund des Patienten ist ungefähr in Höhe der Kniescheibe des Notarztes. In beiden Fällen schiebt die rechte Hand des Intubierenden den Tubus von oben nach unten und gleichzeitig vom Körper des Arztes weg. Diese Bewegung kann durch eine T-Form des Handgriffs der Intubationshilfe unterstützt werden.

Auch kann an einem Abschnitt des Handgriffs, welcher zwischen dem Daumen und dem Zeigefinger angeordnet ist, wenn der Handgriff durch einen Bediener gegriffen ist, ein Abschnitt des Kommunikationsmoduls, insbesondere bei einer LiFi-Kommunikation, angeordnet sein. Dieser Abschnitt des Kommunikationsmoduls kann auch derart von dem Handgriff vorstehen, dass die Schwimmhautfalte zwischen Daumen und Zeigefinger des Bedieners zwischen diesem Abschnitt des Kommunikationsmoduls und dem Handgriff angeordnet werden kann.

Nachzutragen sei noch, dass es ferner denkbar sein kann, eine Intubationshilfe zum Einführen eines Tubus in die Luftröhre eines Patienten bereitzustellen, welche einen länglichen Grundkörper umfasst, wobei der Grundkörper ferner eine Positionierungseinrichtung umfasst, welche dazu eingerichtet ist, eine translatorische Verlagerung eines auf die Intubationshilfe aufgesetzten Tubus in wenigstens einer, insbesondere relativ zu dem Grundkörper translatorischen, Richtung zu begrenzen, und wobei der längliche Grundkörper, insbesondere der Abschnitt der Positionierungseinrichtung davon, mit einer übergeordneten Saugvorrichtung verbindbar ist, unter deren Wirkung Fluid entlang des Grundkörpers aus einem Patienten absaugbar ist. Ist beispielsweise ein Tubus auf den Grundkörper aufgesetzt, so kann der Tubus zusammen mit dem Grundkörper einen entsprechenden Absaugkanal ausbilden. Dabei kann es vorteilhaft sein, wenn der freie Querschnitt des Tubus, welcher zum Absaugen von Fluid verwendbar ist, durch den Grundkörper möglichst minimal ausgefüllt wird.

In einem zweiten (nicht erfindungsgemäßen) Aspekt, welchem dieselbe Aufgabe zu Grunde gelegt ist wie dem ersten Aspekt, wobei diese Aufgabe in dem zweiten (nicht beanspruchten) Aspekt alternativ gelöst wird, betrifft die vorliegende Offenbarung eine Intubationshilfe zum Einführen eines Tubus in die Luftröhre eines Patienten, umfassend einen länglichen Grundkörper, einen sich verjüngenden Abschnitt, welcher an dem einen Längsende des Grundkörpers angeordnet und mit diesem verbunden ist, einen Verbindungsabschnitt, welcher an dem anderen Längsende des Grundkörpers angeordnet und mit diesem verbunden ist, wobei der Verbindungsabschnitt einen ersten Eingriffsabschnitt aufweist, welcher in radialer Richtung im Wesentlichen nach innen weist und dazu eingerichtet ist, mit einer übergeordneten Baugruppe, insbesondere einem Endoskop, in Eingriff zu treten, und einen zweiten Eingriffsabschnitt aufweist, welcher in radialer Richtung im Wesentlichen nach außen weist und dazu eingerichtet ist, mit dem Tubus in Eingriff zu treten, und wobei sowohl der Verbindungsabschnitt als auch der sich verjüngende Abschnitt jeweils einen Aufnahmeabschnitt aufweisen, welche im Wesentlichen als Durchgangsloch ausgebildet sind und dazu eingerichtet sind, jeweils einen Abschnitt der übergeordneten Baugruppe, insbesondere des Endoskops, aufzunehmen.

Der erste Eingriffsabschnitt und/oder der zweite Eingriffsabschnitt des Verbindungsabschnitts kann/können insbesondere dazu eingerichtet sein, mit der übergeordneten Baugruppe und/oder dem Tubus reibschlüssig einzugreifen, so dass zum Beispiel der Tubus auf die Intubationshilfe aufgeschoben werden kann, bis der oben erwähnte Kragen des Tubus durch den Konus der Positionierungseinrichtung bzw. der Halteeinrichtung, wie oben bereits erwähnt, gegriffen wird. Zu diesem Zweck kann der erste und/oder der zweite Eingriffsabschnitt des Verbindungsabschnitts eine diesen Reibschluss unterstützende Beschichtung, beispielsweise aus Gummi, aufweisen. Alternativ oder zusätzlich kann natürlich auch hier eine formschlüssig eingreifende Verriegelungseinheit vorgesehen sein.

Der Grundkörper kann bei der Intubationshilfe des zweiten Aspekts derart ausgebildet sein, dass er, bevor oder nachdem der Tubus auf die Intubationshilfe aufgesetzt worden ist, durch Handkraft des Bedieners in wenigstens einer radialen Richtung, insbesondere nur entlang einer Ebene, in welcher sich eine entsprechende Krümmung erstreckt, gebogen werden kann. Ferner ist es denkbar, die Intubationshilfe, oder wenigstens den Grundkörper der Intubationshilfe, in einer bereits vorgebogenen Ausbildung auszuliefern, so dass, wenn ein Tubus auf den vorgebogenen Grundkörper aufgesteckt wird, der Tubus bereits dadurch in einer gewünschten Weise deformiert wird. Insbesondere für den Fall, dass der Grundkörper nicht dazu eingerichtet sein soll, im Einsatz durch den Bediener gebogen zu werden, kann es vorteilhaft sein, die Intubationshilfe bzw. den Grundkörper in wenigstens zwei unterschiedlichen Varianten bereitzustellen, welche sich in dem Ausmaß der bereits vorhandenen Krümmung des Grundkörpers unterscheiden. Der Grundkörper kann hierzu beispielsweise als ein metallischer Stab ausgebildet sein, welcher den sich verjüngenden Abschnitt mit dem Verbindungsabschnitt, insbesondere an einem Umfangsbereich dieser beiden Abschnitte, verbindet. Auf diese Weise kann der "erste Bereich" der Intubationshilfe gemäß dem ersten Aspekt realisiert werden, ohne hierfür spezielle Bedienungselemente und Seilzüge vorsehen zu müssen.

Die Intubationshilfe bzw. der Grundkörper der Intubationshilfe können in Bezug auf ihre Längserstreckung entsprechend Standardlängen von verfügbaren Beatmungstuben bereitgestellt werden und/oder über einen vorbestimmten Bereich in ihrer Längserstreckung anpassbar sein. Dies kann beispielsweise über eine längenveränderbare Ausgestaltung des Grundkörpers oder aber auch über die in Bezug auf den ersten Aspekt erwähnte verlagerbare Positionierungseinrichtung ausgebildet sein. Somit kann auch bei der Intubationshilfe gemäß dem zweiten Aspekt erreicht werden, dass der sich verjüngende Abschnitt mit einem distalen Ende des auf den Grundkörper aufgesteckten Tubus ausgerichtet werden kann, um Stimmbandverletzungen am Patienten vermeiden zu können.

Der Verbindungsabschnitt kann als ein von dem Grundkörper getrennt hergestelltes und mit diesem lösbar verbundenes Element ausgebildet sein. Somit kann der Verbindungsabschnitt und/oder der Grundkörper als Einwegartikel ausgelegt werden, welche(r) nach einer einmaligen Verwendung zu entsorgen sind/ist. Hierfür kann der Grundkörper an seinem dem Verbindungsabschnitt zugeordneten Ende mit einer geeigneten Verbindungsstruktur, wie beispielsweise einer Halteklammer ausgebildet sein, welche auf eine entsprechende Gegen-Verbindungsstruktur des Verbindungsabschnitts aufgesetzt werden kann.

Es sei explizit darauf hingewiesen, dass der Verbindungsabschnitt und/oder der sich verjüngende Abschnitt mit dem oben erwähnten Kanal zum Absaugen von Aerosol ausgebildet sein können/kann, welches über eine entsprechende Schnittstelle am Verbindungsabschnitt abgeleitet bzw. über diese Sauerstoff durch den Kanal zugeführt werden kann.

Der Aufnahmeabschnitt der Intubationshilfe kann insbesondere auf einen Standarddurchmesser eines verfügbaren Endoskops, beispielsweise eines flexiblen Bronchoskops, ausgelegt sein.

In Bezug auf eine beispielhafte Ausführungsform, kann die Intubationshilfe des zweiten Aspekts auf ein Standard-Endoskop aufgesetzt werden, das heißt das Standard-Endoskop durch die Aufnahmeabschnitte des Verbindungsabschnitts und des sich verjüngenden Abschnitts geführt werden, bis der erste Eingriffsabschnitt mit einem entsprechenden Gegenabschnitt des Endoskops in Eingriff tritt. Auf den, zum Beispiel bereits vorgeformten, Grundkörper wird dann ein Beatmungstubus aufgesteckt. Das freie Ende des Endoskops, welches üblicherweise eine Mechanik zum Krümmen des freien Endes umfasst, kann dann derart über den sich verjüngenden Abschnitt der Intubationshilfe hinaus ragen, dass es die Funktion des "zweiten Bereichs" und des "freien Endes" der Intubationshilfe gemäß dem ersten Aspekt erfüllen kann.

Ferner sei an dieser Stelle darauf hingewiesen, dass die Intubationshilfe gemäß dem ersten Aspekt und/oder die Intubationshilfe gemäß dem zweiten Aspekt wenigstens ein Zwischenstück umfassen kann, welches dazu eingerichtet ist, zwischen dem Tubus und dem Grundkörper angeordnet zu werden, um so auf eine einfache Weise einen Adapter zwischen dem Außendurchmesser der Intubationshilfe zu einem Innendurchmesser eines bestimmten Tubus bereitstellen zu können. Dabei kann es vorteilhaft sein, dass die Intubationshilfe gemäß dem ersten Aspekt und/oder die Intubationshilfe gemäß dem zweiten Aspekt einen Satz an Zwischenstücken umfassen kann, um so eine Mehrzahl von Adaptern für verschiedene Tubus-Innendurchmesser bereitstellen zu können. Ein derartiges Zwischenstück kann als auf den Grundkörper aufsetzbarer Überschaft, beispielsweise in Form einer metallischen Röhre, ausgebildet sein.

Die vorliegende Erfindung wird im Folgenden in größerem Detail mit Bezug auf die begleitenden Zeichnungen beschrieben werden, in welchen
- Figur 1: eine schematische Seitenansicht einer Ausführungsform der erfindungsgemäßen Intubationshilfe gemäß dem ersten Aspekt zeigt;
- Figur 2: eine schematische Seitenansicht auf das freie Ende des Grundkörpers der Intubationshilfe gemäß dem Pfeil II aus Figur 1 zeigt;
- Figur 3: eine schematische Ansicht der Intubationshilfe aus Figur 1 mit daran angeordnetem Tubus und verbundenem Monitor zeigt; und
- Figur 4: eine schematische Ansicht der Intubationshilfe aus Figur 1 mit daran angeordnetem Tubus und direkt angestecktem Monitor zeigt;
- Figur 5a: ein Standard-Endoskop darstellen;
- Figuren 5b und 5c: das Standard-Endoskop aus Figur 5a darstellen, auf welchem eine Intubationshilfe gemäß dem zweiten (nicht erfindungsgemäßen)Aspekt angeordnet ist,
- Figur 6: die Intubationshilfe gemäß dem zweiten (nicht erfindungsgemäßen) Aspekt in einer schematischen Seitenquerschnittsansicht zeigt;
- Figur 7: eine schematische Seitenquerschnittsansicht der Intubationshilfe gemäß dem zweiten (nicht erfindungsgemäßen) Aspekt darstellt.

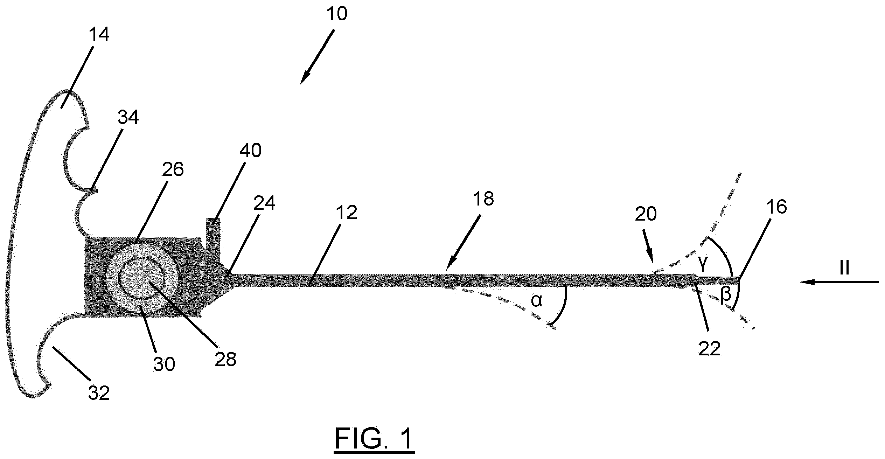

In Figur 1 ist eine erfindungsgemäße Intubationshilfe allgemein mit dem Bezugszeichen 10 bezeichnet. Die Intubationshilfe 10 umfasst einen länglichen Grundkörper 12, welcher an seinem einen Ende einen Handgriff 14 und an seinem anderen Ende ein freies Ende 16 aufweist. Der Grundkörper 12 ist dazu eingerichtet, in einem ersten Bereich 18 derart gekrümmt zu werden, dass ein Abschnitt des Grundkörpers 12, welcher zwischen dem freien Ende 16 und dem ersten Bereich 18 angeordnet ist, in Figur 1 nach unten um bis zu einem Winkel α ausgelenkt werden kann. Ferner weist der Grundkörper 12 einen zweiten Bereich 20 auf, welcher dazu eingerichtet ist, derart gekrümmt zu werden, dass das freie Ende 16 relativ zu einem Abschnitt zwischen dem ersten Bereich 18 und dem zweiten Bereich 20 bis zu einem Winkel β, welcher sich in der gleichen Richtung wie der Winkel α erstreckt oder, in der entgegengesetzten Richtung, um bis zu einem Winkel γ ausgelenkt werden kann.

Zwischen dem freien Ende 16 und dem zweiten Bereich 20 des Grundkörpers 12 ist ein sich verjüngender Abschnitt 22 angeordnet, über welchen sich ein Außendurchmesser des Grundkörpers 12 im Abschnitt des zweiten Bereichs 20 zu einem Außendurchmesser des Grundkörpers 12 im Abschnitt des freien Endes 16 allmählich reduziert.

An seinem dem freien Ende 16 entgegengesetzten Ende weist der längliche Grundkörper 12 eine Positionierungseinrichtung 24 auf, welche hier als ein Konus 24 ausgebildet ist. In einem Abschnitt zwischen dem Konus 24 und dem Handgriff 14 ist eine Bedienungseinrichtung 26 angeordnet, welche ein erstes Bedienungselement 28 und ein zweites Bedienungselement 30 umfasst. Das erste Bedienungselement 28 ist mit dem ersten Bereich 18 über einen Seilzug, wie beispielsweise einen Bowdenzug, derart gekoppelt, dass eine Betätigung des ersten Bedienungselements 28 durch einen Bediener in einer entsprechenden Richtung eine Auslenkung des ersten Bereichs 18 gemäß der Eingabemenge an dem ersten Bedienungselement 28 hervorruft. In einer analogen Weise ist das zweite Bedienungselement 30 mit dem zweiten Bereich 20 gekoppelt.

Das erste Bedienungselement 28 ist mit einer Rasteinrichtung verbunden, so dass über das Bedienungselement 28 selbst oder beispielsweise über einen entsprechenden Abschnitt des Handgriffs 14 eine eingestellte Auslenkung des ersten Bereichs 18 arretiert werden kann.

Wie in Figur 1 zu sehen, umfasst der Handgriff 14 eine Einfinger-Aufnahme 32, welche insbesondere dazu eingerichtet ist, einen Zeigefinger eines Bedieners aufzunehmen, und welche an einer gleichen Seite der Intubationshilfe 10 angeordnet ist, an welcher der erste Bereich um bis zu dem Winkel α und der zweite Bereich um bis zu dem Winkel β ausgelenkt werden kann, und eine Zweifinger-Aufnahme 34, welche dazu eingerichtet ist, insbesondere den Mittelfinger und den Ringfinger des Bedieners aufzunehmen.

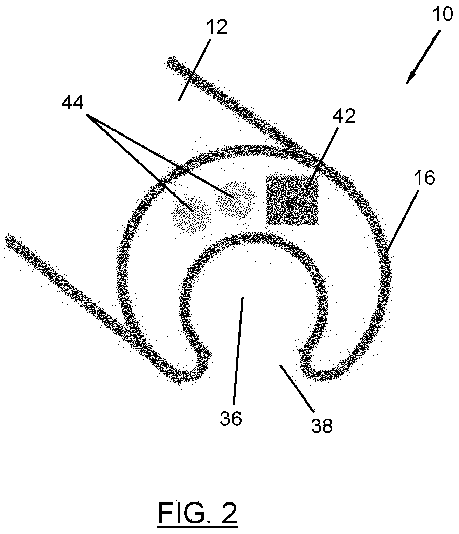

Figur 2 zeigt eine Ansicht des freien Endes 16 der erfindungsgemäßen Intubationshilfe 10 aus Figur 1, welche durch den Pfeil II in Figur 1 angezeigt ist. Dabei ist zu erkennen, dass in dem Grundkörper 12 ein Kanal 36 angeordnet ist, welcher entlang der Längserstreckung des Grundkörpers 12 ausgebildet ist. Dabei ist der Kanal 36 nicht vollständig innerhalb des Grundkörpers 12 angeordnet, sondern steht entlang seine Länge, in der hier dargestellten Ausführungsform von dem freien Ende 16 bis zu dem Konus 24, über eine Unterbrechung in der Umfangsrichtung des Kanals 36 mit einer Außenseite des Grundkörpers 12 in Fluidverbindung. Somit entsteht ein Spalt 38, welcher sich in dieser Ausführungsform von dem freien Ende 16 bis zu dem Konus der Positionierungseinrichtung 24 erstreckt, wobei der Kanal 36 über diesen Spalt 38 beispielsweise für eine Reinigung zugänglich ist. Wie weiter oben beschrieben, kann es aber auch denkbar sein, dass der Kanal 36 an dem sich verjüngenden Abschnitt 22 des Grundkörpers 12 endet.

Der Kanal 36 ist an seinem dem freien Ende 16 entgegengesetzten Ende mit einer Schnittstelle 40 (siehe Figur 1) verbunden, welche dazu eingerichtet ist, mit einer Saugvorrichtung und/oder mit einer Gasquelle und/oder mit einem Viren-/Bakterienfilter oder Ähnlichem verbunden zu werden. Unter der Wirkung der Saugvorrichtung, welche an die Schnittstelle 40 angeschlossen ist, kann beim Intubieren eines Patienten Aerosol aus dem Mund- /Rachenraum, dem Kehlkopf und der Luftröhre des Patienten abgesaugt werden, so dass ein freier Austritt des potentiell virenbelasteten Aerosols in eine Umgebung verhindert werden kann. Der Kanal 36 kann auch dazu verwendet werden, beispielsweise Sauerstoff aus einer Gasquelle über die Schnittstelle 40 in den Patienten einzuleiten.

Ferner ist in Figur 2 zu erkennen, dass an dem freien Ende 16 des Grundkörpers 12 eine Bildgebungseinheit 42, welche dazu eingerichtet ist, Bildinformationen in einer Umgebung des freien Endes 16 der Intubationshilfe 10 aufzunehmen, sowie eine Beleuchtungsvorrichtung 44 angeordnet ist, welche hier als zwei LED-Lichtquellen 44 ausgebildet ist.

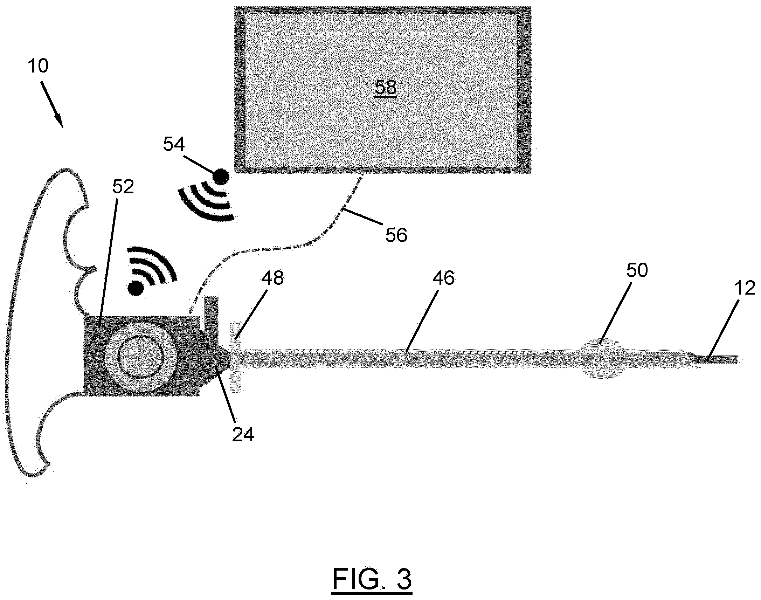

In Figur 3 ist die Intubationshilfe 10 aus Figur 1 gezeigt, wobei auf dem Grundkörper 12 ein Beatmungstubus 46 aufgesetzt ist. Der Beatmungstubus 46 weist einen Kragen 48 auf, welcher, nachdem der Tubus 46 in einen Patienten eingesetzt und die Intubationshilfe 10 aus dem Tubus 46 und dem Patienten entfernt worden ist, als Schnittstelle zu einer Beatmungsvorrichtung fungiert. Im Bereich des Kragens 48 ist der Tubus 46 über den Konus 24, welcher hier als Halteeinrichtung dient, reibschlüssig gegenüber einer bestimmten Kraft erhalten, welche den Tubus 46 von dem Grundkörper 12 (in Figur 3 nach rechts) verlagert. Der Beatmungstubus 46 weist ferner einen erweiterbaren Bereich 50 auf, welcher beispielsweise mit einem Gas befüllt werden kann, um, sobald sich der Tubus 46 an seiner gewünschten Stelle in dem Patienten befindet, eine Abdichtung eines mit der Lunge verbundenen Luftraums gegenüber einem mit dem Mund verbundenen Luftraum zu erreichen.

Die Intubationshilfe 10 umfasst ein Kommunikationsmodul 52, welches dazu eingerichtet ist, drahtlos 54 und/oder drahtgebunden 56 mit einer externen Einheit 58 zu kommunizieren, welche hier ein tragbarer Monitor ist, welcher dazu eingerichtet ist, die Bilddaten anzuzeigen, welche über die Bildgebungseinheit 42 aufgenommen worden sind.

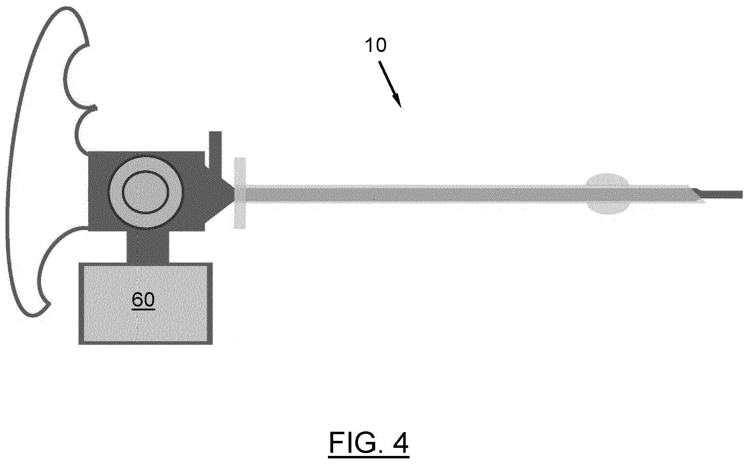

In Figur 4 ist dargestellt, dass an der Intubationshilfe 10, alternativ oder zusätzlich zu der Kopplung der Intubationshilfe 10 mit einem externen Monitor gemäß Figur 3, an einem Abschnitt, an welchem insbesondere die Bedienungseinrichtung 26 angeordnet ist, direkt ein Monitor 60 angeordnet werden kann. Natürlich kann die Intubationshilfe 10 auch derart ausgebildet sein, dass diese sowohl dem Monitor 58 (Figur 3) als auch mit dem Monitor 60 (Figur 4) gleichzeitig kommunizieren kann.

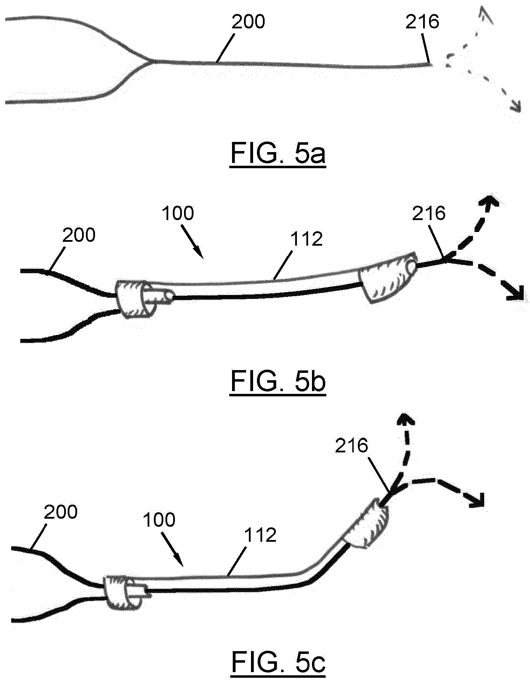

In Figuren 5a bis 5c ist ein Standard-Endoskop 200 dargestellt, welches an seinem freien Ende 216 über eine entsprechende Abwinkelungsmechanik verfügt, wobei in den Figuren 5b und 5c eine Intubationshilfe 100 gemäß einem zweiten (nicht erfindungsgemäßen) Aspekt der vorliegenden Offenbarung aufgesetzt ist (von welcher hier aus Gründen der Übersichtlichkeit lediglich ein Grundkörper 112 dargestellt ist). Zu der Intubationshilfe 10 gemäß dem ersten Aspekt ähnliche Elemente der Intubationshilfe 100 gemäß dem zweiten Aspekt sind dabei mit gleichen Bezugszeichen versehen, jedoch erhöht um 100.

Die Figuren 5b und 5c zeigen dabei zwei Grundkörper 112, welche zueinander unterschiedliche Vorbiegungen aufweisen, welche einer Krümmungsfunktionalität des "ersten Bereichs" des ersten Aspekts dieser Offenbarung entsprechen.

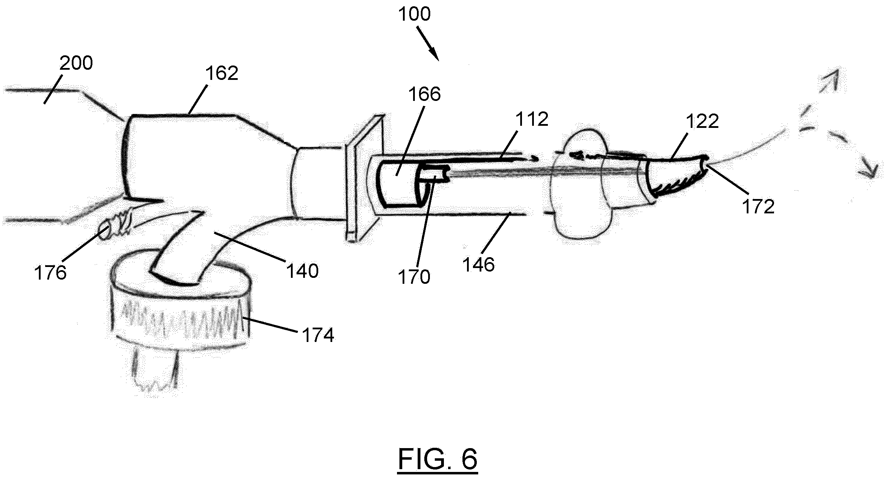

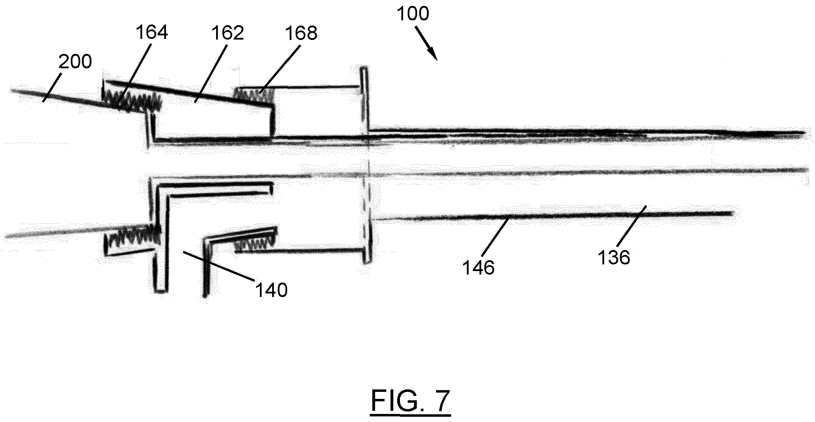

In Figur 6 ist die Intubationshilfe 100 in einer schematischen Seitenansicht gezeigt. Dabei ist zu erkennen, dass auf das Endoskop 200 die Intubationshilfe 100 so weit aufgesetzt ist, bis ein Verbindungsabschnitt 162, genauer gesagt, in der dargestellten Ausführungsform, ein erster Eingriffsabschnitt 164 (siehe Figur 7), mit dem Endoskop 200 in Eingriff tritt.

An dem Verbindungsabschnitt 162 ist über eine Halteklammer 166 ein Grundkörper 112 angebracht, welcher an seinem der Halteklammer 166 entgegengesetzten Ende einen sich verjüngenden Abschnitt 122 aufweist. Auf den Grundkörper 112 ist ein Beatmungstubus 146 derart aufgesetzt, dass er an dem Verbindungsabschnitt 162 über einen geeigneten zweiten Eingriffsabschnitt 168 (siehe Figur 7) gegenüber einer Lösekraft gehalten wird, welche entgegen einer Aufsteckrichtung des Beatmungstubus 164 auf die Intubationshilfe 100 wirkt.

**In** Figur 6 ist ferner zu erkennen, dass ein distaler Abschnitt des Endoskops 200 durch jeweilige Aufnahmeabschnitte 170 und 172 des Verbindungsabschnitts 162 und des sich verjüngenden Abschnitts 122 hindurch geführt ist.

**In** Figur 7 ist eine schematische Seitenquerschnittsansicht der Intubationshilfe 100 dargestellt. Wie hier zu erkennen ist, wird durch den aufgesetzten Tubus 146 ein Kanal 136 in dem Inneren des Tubus 146 ausgebildet, welcher zum Absaugen von Aerosol über eine mit der Schnittstelle 140 des Verbindungsabschnitts 162 verbundene Saugvorrichtung verwendet werden kann. Zurückgehend zu Figur 6, ist zu sehen, dass neben der Schnittstelle 140, welche mit einem Viren-/Bakterienfilter 174 verbunden ist, an dem Verbindungsabschnitt 162 ferner eine von der Schnittstelle 140 getrennt ausgebildete Schnittstelle 176 vorgesehen ist, über welche Sauerstoff in den Kanal 136 eingeleitet werden kann.

## Patentansprüche

1. Intubationshilfe (10) zum Einführen eines Tubus (46) in die Luftröhre eines Patienten,
wobei die Intubationshilfe (10) einen länglichen Grundkörper (12) umfasst, welcher an seinem einen Ende mit einem Handgriff (14) verbunden ist und an seinem anderen Ende ein freies Ende (16) aufweist, wobei die Intubationshilfe (10), insbesondere der Handgriff (14), mit einer Bedienungseinrichtung (26) bereitgestellt ist,
wobei der Grundkörper (12) dazu eingerichtet ist, auf eine Betätigung der Bedienungseinrichtung (26) hin, in einem ersten Bereich (18) des Grundkörpers (12) gekrümmt zu werden,
wobei der Grundkörper (12) ferner dazu eingerichtet ist, auf eine weitere Betätigung der Bedienungseinrichtung (26) hin, in einem zweiten Bereich (20) des Grundkörpers (12) gekrümmt zu werden, welcher von dem ersten Bereich (18) getrennt und dem freien Ende (16) des Grundkörpers (12) benachbart angeordnet ist, und
wobei der Grundkörper (12) ferner eine Positionierungseinrichtung (24) umfasst, welche dazu eingerichtet ist, eine translatorische Verlagerung eines auf die Intubationshilfe (10) aufgesetzten Tubus (46) in wenigstens einer Richtung zu begrenzen,
**dadurch gekennzeichnet, dass**
der Grundkörper (12) einen Kanal (36) umfasst, welcher sich zumindest teilweise parallel zu einer Längsachse des Grundkörpers (12) erstreckt, und welcher dazu geeignet ist, Fluid aufzunehmen und das Fluid zu einem dem Handgriff (14) zugeordneten Ende (40) des Kanals (36) zu leiten,
wobei mit dem dem Handgriff (14) zugeordneten Ende (40) des Kanals (36) wahlweise verbindbar ist
eine Saugeinheit und ein Viren- und Bakterienfilter, welcher dazu geeignet ist, Viren und Bakterien aus dem von dem Patienten angesaugten Fluid zu filtern, oder
eine Gasquelle, insbesondere eine Sauerstoffquelle, welche dazu eingerichtet ist, Sauerstoff über den Kanal (36) an den Patienten abzugeben, und
eine Wandung des Grundkörpers (12), in Umfangsrichtung des länglichen Grundkörpers (12) betrachtet, derart unterbrochen ausgebildet ist, dass ein sich in Längsrichtung des Grundkörpers (12) erstreckender Spalt (38) gebildet ist, welcher den Kanal (36) mit einer den Grundkörper (12) umgebenden Außenseite verbindet.

2. Intubationshilfe (10) nach Anspruch 1,
**dadurch gekennzeichnet, dass** die Positionierungseinrichtung (24) eine Halteeinrichtung umfasst, welche dazu eingerichtet ist, mit einem Abschnitt des Tubus (46) einzugreifen, um diesen, zumindest translatorisch, relativ zu dem Grundkörper (12) zu sichern, wobei die Halteeinrichtung insbesondere als ein dem Handgriff (14) benachbart angeordneter Konus (24), welcher mit einem Abschnitt des Tubus (46) reibschlüssig in Eingriff tritt, und/oder als eine Verriegelungseinheit ausgebildet ist, welche mit dem Abschnitt des Tubus (46) formschlüssig in Eingriff tritt.

3. Intubationshilfe (10) nach einem der vorhergehenden Ansprüche,
**dadurch gekennzeichnet, dass** die Intubationshilfe (10) ein Zwischenstück umfasst, welches dazu eingerichtet ist, zwischen dem Tubus (46) und dem Grundkörper (12) angeordnet zu werden.

4. Intubationshilfe (10) nach einem der vorhergehenden Ansprüche,
**dadurch gekennzeichnet, dass** der Grundkörper (12) einen sich verjüngenden Abschnitt (22) aufweist, welcher dem freien Ende (16) des Grundkörpers (12) benachbart, insbesondere zwischen dem freien Ende (16) des Grundkörpers (12) und dem zweiten krümmbaren Bereich (20) des Grundkörpers (12), angeordnet ist.

5. Intubationshilfe (10) nach Anspruch 1 und Anspruch 4,
**dadurch gekennzeichnet, dass** sich der Kanal (36) von dem dem Handgriff (14) zugeordneten Ende (40) bis zu dem sich verjüngenden Abschnitt (22) des Grundkörpers (12) erstreckt.

6. Intubationshilfe (10) nach einem der vorhergehenden Ansprüche,
**dadurch gekennzeichnet, dass** an dem freien Ende (16) des Grundkörpers (12) eine Bildgebungseinheit (42), insbesondere ein Bildsensor, angeordnet ist, welche dazu eingerichtet ist, Bilddaten aufzunehmen und diese weiterzuleiten.

7. Intubationshilfe (10) nach einem der vorhergehenden Ansprüche,
**dadurch gekennzeichnet, dass** an dem freien Ende (16) des Grundkörpers (12) eine Beleuchtungsvorrichtung (44) angeordnet ist, welche dazu eingerichtet ist, Licht, insbesondere an den durch die Bildgebungseinheit (42) aufzunehmenden Bereich, zu emittieren.

8. Intubationshilfe (10) nach einem der vorhergehenden Ansprüche,
**dadurch gekennzeichnet, dass** der erste krümmbare Bereich (18) in einem Abstand zu dem freien Ende (16) des Grundkörpers (12) von 10 cm bis 20 cm, insbesondere bei etwa 15 cm, angeordnet ist.

9. Intubationshilfe (10) nach einem der vorhergehenden Ansprüche,
**dadurch gekennzeichnet, dass** Ebenen, in welchen der Grundkörper (12) in dem ersten Bereich (18) und in dem zweiten Bereich (20) krümmbar sind, im Wesentlichen parallel zueinander, insbesondere zusammenfallend sind.

10. Intubationshilfe (10) nach einem der vorhergehenden Ansprüche,
**dadurch gekennzeichnet, dass** der erste Bereich (18) von 0° bis 30° krümmbar ist und/oder der zweite Bereich (20) von -60° bis 45° krümmbar ist.

11. Intubationshilfe (10) nach einem der vorhergehenden Ansprüche,
**dadurch gekennzeichnet, dass** die Bedienungseinrichtung (26) wenigstens ein erstes Bedienungselement (28) und ein zweites Bedienungselement (30) umfasst, wobei das erste Bedienungselement (28) für eine Auslenkung des ersten Bereichs (18) des Grundkörpers (12) eingerichtet ist und das zweite Bedienungselement (30) für eine Auslenkung des zweiten Bereichs (20) des Grundkörpers (12) eingerichtet ist, und
wobei dem ersten Bedienungselement (28) eine Rasteinrichtung zugeordnet ist, welche dazu eingerichtet ist, auf eine Betätigung des ersten Bedienungselements (28) um wenigstens ein vorbestimmtes Ausmaß hin, eine Rückwärtsbewegung des ersten Bedienungselements (28), entgegen der Betätigungsrichtung davon, zu verhindern.

12. Intubationshilfe (10) nach einem der vorhergehenden Ansprüche,
**dadurch gekennzeichnet, dass** die Intubationshilfe (10) ein Kommunikationsmodul (52), insbesondere ein drahtloses Kommunikationsmodul (52), umfasst, welches dazu eingerichtet ist, mit einer externen Einheit (58, 60) zu kommunizieren, wobei das Kommunikationsmodul (52) insbesondere in dem Handgriff (14) der Intubationshilfe (10) angeordnet ist.

## Claims

1. Intubation aid (10) for inserting a tube (46) into a patient's trachea,
wherein the intubation aid (10) comprises an elongate main part (12) which at its one end is connected to a handle (14) and at its other end has a free end (16), wherein the intubation aid (10), in particular the handle (14), is provided with an operating device (26),
wherein the main part (12) is designed to be bent in a first region (18) of the main part (12) when the operating device (26) is actuated,
wherein the main part (12) is furthermore designed, upon further actuation of the operating device (26), to be bent in a second region (20) of the main part (12), which is separate from the first region (18) and is arranged adjacent to the free end (16) of the main part (12), and
wherein the main part (12) further comprises a positioning device (24) which is designed to limit a translational displacement of a tube (46) fitted onto the intubation aid (10) in at least one direction,
**characterized in that** the main part (12) comprises a channel (36) which extends parallel, at least in part, to a longitudinal axis of the main part (12) and which is suitable for receiving fluid and for conducting the fluid to an end (40) of the channel (36) assigned to the handle (14),
wherein one of the following is selectively connectable to the end (40) of the channel (36) assigned to the handle (14)
a suction unit and a virus and bacteria filter suitable for filtering viruses and bacteria from the fluid suctioned out from the patient, or
a gas source, in particular an oxygen source designed to deliver oxygen to the patient via the channel (36), and
**in that** a wall of the main part (12), viewed in the circumferential direction of the elongate main part (12), is designed interrupted such that a gap (38) extending in the longitudinal direction of the main part (12) is formed and connects the channel (36) to an exterior surrounding the main part (12).

2. Intubation aid (10) according to claim 1,
**characterized in that** the positioning device (24) comprises a holding device which is designed to engage with a segment of the tube (46) in order to secure the latter, at least translationally, relative to the main part (12), in particular wherein the holding device is a cone (24) that is arranged adjacent to the handle (14) and that engages a segment of the tube (46) in a friction fit, and/or is embodied as a locking unit which engages the segment of the tube (46) in a positive fit.

3. Intubation aid (10) according to any of the preceding claims,
**characterized in that** the intubation aid (10) comprises an intermediate piece designed to be arranged between the tube (46) and the main part (12).

4. Intubation aid (10) according to one of the preceding claims,
**characterized in that** the main part (12) has a tapering segment (22) which is arranged adjacent to the free end (16) of the main part (12), in particular between the free end (16) of the main part (12) and the second bendable region (20) of the main part (12).

5. Intubation aid (10) according to claim 1 and claim 4,
**characterized in that** the channel (36) extends from the end (40) associated with the handle (14) to the tapering segment (22) of the main part (12).

6. Intubation aid (10) according to one of the preceding claims,
**characterized in that** an imaging unit (42), in particular an image sensor, which is designed to record and forward imaging data, is arranged on the free end (16) of the main part (12).

7. Intubation aid (10) according to one of the preceding claims,
**characterized in that** an illumination device (44), which is designed to emit light, in particular onto the region to be recorded by the imaging unit (42), is arranged at the free end (16) of the main part (12).

8. Intubation aid (10) according to one of the preceding claims,
**characterized in that** the first bendable area (18) is arranged at a distance of 10 cm to 20 cm, in particular about 15 cm, from the free end (16) of the main part (12).

9. Intubation aid (10) according to one of the preceding claims,
**characterized in that** planes in which the main part (12) can be curved in the first region (18) and in the second region (20) are essentially parallel to one another, in particular coinciding.

10. Intubation aid (10) according to one of the preceding claims,
**characterized in that** the first region (18) can be bent from 0° to 30° and/or the second region (20) can be bent from -60° to 45°.

11. Intubation aid (10) according to one of the preceding claims,
**characterized in that** the operating device (26) comprises at least a first operating element (28) and a second operating element (30), wherein the first operating element (28) is designed for deflecting the first region (18) of the main part (12) and the second operating element (30) is designed for deflecting the second region (20) of the main part (12), and
wherein the first operating element (28) is assigned a latching device designed for preventing rearward movement of the first operating element (28), counter to the actuating direction thereof, upon actuation of the first operating element (28) by at least a predetermined amount.

12. Intubation aid (10) according to one of the preceding claims, **characterized in that** the intubation aid (10) comprises a communication module (52), in particular a wireless communication module (52), which is designed to communicate with an external unit (58, 60), wherein the communication module (52) is arranged in particular in the handle (14) of the intubation aid (10).

## Revendications

1. Aide à l'intubation (10) pour introduire un tube (46) dans la trachée d'un patient, dans lequel l'aide à l'intubation (10) comprend un corps de base allongé (12) qui est relié à une extrémité à une poignée (14) et qui présente à son autre extrémité une extrémité libre (16), dasn lequel l'aide à l'intubation (10), en particulier la poignée (14), est pourvue d'un dispositif de commande (26),
dans lequel le corps de base (12) est conçu pour être courbé dans une première zone (18) du corps de base (12) lors de l'actionnement du dispositif de commande (26),
dans lequel le corps de base (12) est en outre conçu pour être courbé dans une deuxième zone (20) du corps de base (12) lors d'un autre actionnement du dispositif de commande (26), laquelle zone est séparée de la première zone (18) et est disposée à proximité de l'extrémité libre (16) du corps de base (12), et
dans lequel le corps de base (12) comprend en outre un dispositif de positionnement (24) qui est conçu pour limiter un déplacement en translation d'un tube (46) placé sur l'aide à l'intubation (10) dans au moins une direction,
**caractérisé en ce que**
le corps de base (12) comprend un canal (36) qui s'étend au moins en partie parallèlement à un axe longitudinal du corps de base (12) et qui est adapté pour recevoir du fluide et pour diriger le fluide vers une extrémité (40) du canal (36) associée à la poignée (14), l'extrémité (40) du canal (36) associée à la poignée (14) pouvant être reliée au choix à une unité d'aspiration et à un filtre anti-virus et anti-bactéries qui est adapté pour filtrer les virus et les bactéries du fluide aspiré par le patient, ou une source de gaz, en particulier une source d'oxygène, qui est conçue pour délivrer de l'oxygène au patient via le canal (36), et
une paroi du corps de base (12), vu dans la direction périphérique du corps de base allongé (12), est réalisée de manière interrompue de telle sorte qu'une fente (38) s'étendant dans la direction longitudinale du corps de base (12) est formée, laquelle relie le canal (36) à une face extérieure entourant le corps de base (12).

2. Aide à l'intubation (10) selon la revendication 1,
**caractérisée en ce que** le dispositif de positionnement (24) comprend un dispositif de retenue qui est conçu pour s'engager avec une partie du tube (46) afin de le fixer, au moins en translation, par rapport au corps de base (12), dans lequel le dispositif de maintien est notamment conçu sous la forme d'un cône (24) disposé adjacent à la poignée (14) et s'engageant par friction avec une partie du tube (46), et/ou conçu sous la forme d'une unité de verrouillage s'engageant par complémentarité de forme avec la partie du tube (46).

3. Aide à l'intubation (10) selon l'une des revendications précédentes,
**caractérisée en ce que** l'aide à l'intubation (10) comprend une pièce intermédiaire qui est conçue pour être disposée entre le tube (46) et le corps de base (12).

4. Aide à l'intubation (10) selon l'une des revendications précédentes,
**caractérisé en ce que** le corps de base (12) présente une partie effilée (22) qui est disposée adjacente à l'extrémité libre (16) du corps de base (12), en particulier entre l'extrémité libre (16) du corps de base (12) et la deuxième zone courbable (20) du corps de base (12).

5. Aide à l'intubation (10) selon la revendication 1 et la revendication 4,
**caractérisée en ce que** le canal (36) s'étend de l'extrémité (40) associée à la poignée (14) jusqu'à la partie effilée (22) du corps de base (12).

6. Aide à l'intubation (10) selon l'une des revendications précédentes,
**caractérisée en ce qu'**une unité d'imagerie (42), en particulier un capteur d'images, est disposée à l'extrémité libre (16) du corps de base (12), laquelle est conçue pour enregistrer des données d'images et les transmettre.

7. Aide à l'intubation (10) selon l'une des revendications précédentes,
**caractérisée en ce qu'**un dispositif d'éclairage (44) est disposé à l'extrémité libre (16) du corps de base (12), lequel est conçu pour émettre de la lumière, en particulier vers la zone à enregistrer par l'unité d'imagerie (42).

8. Aide à l'intubation (10) selon l'une des revendications précédentes,
**caractérisée en ce que** la première zone courbable (18) est disposée à une distance de l'extrémité libre (16) du corps de base (12) comprise entre 10 cm et 20 cm, en particulier à environ 15 cm.

9. Aide à l'intubation (10) selon l'une des revendications précédentes,
**caractérisée en ce que** les plans dans lesquels le corps de base (12) peut être courbé dans la première zone (18) et dans la deuxième zone (20) sont essentiellement parallèles entre eux, en particulier coïncidents.

10. Aide à l'intubation (10) selon l'une des revendications précédentes,
**caractérisée en ce que** la première zone (18) est courbable de 0° à 30° et/ou la deuxième zone (20) est courbable de -60° à 45°.

11. Aide à l'intubation (10) selon l'une des revendications précédentes,
**caractérisé en ce que** le dispositif de commande (26) comprend au moins un premier élément de commande (28) et un deuxième élément de commande (30), dans lequel le premier élément de commande (28) est conçu pour déplacer la première zone (18) du corps de base (12) et le deuxième élément de commande (30) est conçu pour déplacer la deuxième zone (20) du corps de base (12), et
dans lequel un dispositif d'encliquetage est associé au premier élément de commande (28), lequel dispositif d'encliquetage est conçu pour empêcher, lors d'un actionnement du premier élément de commande (28) d'au moins une amplitude prédéterminée, un mouvement vers l'arrière du premier élément de commande (28) dans le sens opposé au sens d'actionnement.

12. Aide à l'intubation (10) selon l'une des revendications précédentes,
**caractérisée en ce que** l'aide à l'intubation (10) comprend un module de communication (52), en particulier un module de communication sans fil (52), qui est conçu pour communiquer avec une unité externe (58, 60),
dasns lequel le module de communication (52) est notamment disposé dans la poignée (14) de l'aide à l'intubation (10).
